Europäisches Patentamt

European Patent Office

Office européen des brevets

(11) Publication number: **0 147 194**
**B1**

## (12) EUROPEAN PATENT SPECIFICATION

(45) Date of publication of patent specification: **19.07.89**

(21) Application number: **84308976.4**

(22) Date of filing: **20.12.84**

(51) Int. Cl.⁴: **C 07 K 7/44, A 61 K 37/02**

(54) Compositions for treating cerebral ischemia.

(30) Priority: **22.12.83 US 564741**

(43) Date of publication of application:
**03.07.85 Bulletin 85/27**

(45) Publication of the grant of the patent:
**19.07.89 Bulletin 89/29**

(84) Designated Contracting States:
**AT BE CH FR GB IT LI LU NL SE**

(56) References cited:
**EP-A-0 029 300**
**EP-A-0 096 592**
**EP-A-0 124 243**
**NATURE, vol. 287, no. 5784, October 23, 1980,
pp. 678-679, Macmillan Journals Ltd.,
Chesham, Bucks, GB, M.J. BROWNSTEIN:
"Opioid peptides: Search for the precursors"**
**CHEMICAL ABSTRACTS, vol. 95, no. 11,
September 14, 1981, p. 709, ref. no. 98301h;
Columbus, Ohio, US, N. MINAMINO et al.:
"Endogenous "big" Leu-enkephalins from
porcine hypothalamus: Purifications,
structures and syntheses of alpha-, beta-
neoendorphin and PH-8P."**

(73) Proprietor: **Hosobuchi, Yoshio**
**116 Kinross Drive**
**San Rafael California 94901 (US)**
(73) Proprietor: **Lee, Nancy M.**
**1839 Funston Avenue**
**San Francisco California 94116 (US)**
(73) Proprietor: **Loh, Horace H.**
**54 Mendosa**
**San Francisco California 94116 (US)**
(73) Proprietor: **Chang, Jaw-Kang**
**90 Curtis Court**
**San Carlos California 94070 (US)**

(72) Inventor: **Hosobuchi, Yoshio**
**116 Kinross Drive**
**San Rafael California 94901 (US)**
Inventor: **Lee, Nancy M.**
**1839 Funston Avenue**
**San Francisco California 94116 (US)**
Inventor: **Loh, Horace H.**
**54 Mendosa**
**San Francisco California 94116 (US)**
Inventor: **Chang, Jaw-Kang**
**90 Curtis Court**
**San Carlos California 94070 (US)**

(56) References cited:
**NATURE, vol. 312, no. 5994, December 6, 1984,
pp. 551-552, Macmillan Journals Ltd.,
Chesham, Bucks, GB, D.S. BASKIN et al.:
"Dynorphin(1-13) improves survival in cats
with focal cerebral ischaemia"
Chemical Abstracts, vol. 102, 160755c**

(74) Representative: **Paget, Hugh Charles Edward
et al
MEWBURN ELLIS & CO. 2/3 Cursitor Street
London EC4A 1BQ (GB)**

## Description

The present invention generally relates to the use of an opioid peptide, and more particularly of dynorphin for the manufacture of an agent against cerebral ischemia or an agent for prolonging survival of a patient suffering from acute focal cerebral ischemia. It relates also to compositions useful for such treatments and processes of preparing the compositions.

While many infectious diseases have been controlled or eliminated by medical science, chronic diseases—such as heart attack, stroke and cancer—have emerged as major causes of death. Where death does not result from stroke, the victim is often seriously disabled. The death rate for stroke victims is over 2 per 1,000 in the United States. The Japanese, while having one the lowest incidences of death from heart disease, have one of the highest from strokes.

Various different compounds have been suggested as useful for the treatment of stroke. For example, it appears that aspirin may reduce the risk of transient ischemic attacks, or small strokes, and deaths from stroke. U.S. Patent 4,256,883, inventors Nicolaou et al., issued March 17, 1981, discloses prostacyclin analogs said to be useful in vascular constrictions and in cerebral strokes associated with essential hypertension. U.S. Patent 4,364,951, inventors Skuballa et al., issued December 21, 1982, discloses prostacyclins said to possess properties useful, *inter alia*, in treating stroke. U.S. Patent 4,394,385, inventor Cragoe, issued July 19, 1983, discloses the use of benzofuranyloxyacetic acids and anti-inflammatory steroids said to be useful in controlling edema from ischemic stroke.

It has been recently reported that the opiate antagonist naloxone can reverse neurologic deficits secondary to cerebral ischemia, whereas morphine exacerbates them. Baskin and Hosobuchi, "Naloxone reversal of ischaemic neurological deficits in man", *Lancet* 2:272—275 (1981). It has also been reported that neurologic deficits produced by unilateral carotid ligation in gerbils can be reversed by the intraperitoneal administration of naloxone. Hosobuchi et al., "Reversal of induced ischemic neurologic deficit in gerbils by the opiate antagonist naloxone", *Science* 215:69—71 (1982).

However, Levy, et al. have reported that treatment with naloxone did not produce improved neurologic function or alter infarct size in gerbils that had undergone temporary carotid occlusion. ("Failure of naloxone to limit clinical or morphological brain damage in gerbils with unilateral carotid artery occlusion", *Abstracts of the 12th Annual Meeting of the Society for Neuroscience*, p. 248 (1982).) Similarly, Holaday and D'Amato reported that naloxone had no beneficial effect on either survival or neurologic function in several different models of stroke in gerbils. ("Naloxone or TRH fails to improve neurologic deficits in gerbil models of 'stroke'," *Life Science* 31:385—392 (1982).)

EP—A—96592 discloses amidated dynorphins (1—10) to (1—12) for potentiating narcotic or peptide induced analgesia in tolerant hosts.

The present addressed problem is that of providing agents for the treatment of patients suffering from cerebral ischemia.

In the invention an opioid peptide is used for the production of an agent against cerebral ischemia or an agent for prolonging survival of a patient suffering from acute focal cerebral ischemia, the opioid peptide having the amino acid sequence

$$\text{TYR-GLY-GLY-PHE-LEU-ARG-ARG-AA}^8\text{-AA}^9\text{-AA}^{10}\text{-(AA}^{11})_w,$$

wherein $AA^8$ is TYR, ILE, LEU or LYS, $AA^9$ is ARG or PRO, $AA^{10}$ is PRO or LYS, $AA^{11}$ is LYS, LYS-LEU or LYS-LEU-LYS, w is 0 or 1, and being in acid or amidated form.

Particularly preferred opioid peptides are dynorphin (1—13) and dynorphin (1—10) amide.

A method of treating a patient suffering from cerebral ischemia thus comprises administering to the patient a therapeutically effective dose of the agent following the cerebral ischemia. Subsequent doses are then preferably delivered to the patient. Practice of this treatment with patients suffering from acute focal cerebral ischemia is useful in prolonging survival, and is believed to be useful in partially reversing neurologic deficits resulting from cerebral ischemia.

### Best mode of practicing the invention

Since the discovery of opiate receptors within the central nervous system, it has been believed that endogenous opiate ligands may be involved in the central nervous system function in both health and disease states. Investigate attention has focused primarily on the role of these substances in the modulation of the perception of painful stimuli, but they have also been implicated in pituitary function, seizure disorders and mental illness.

Opioid peptides are found in the circulatory system, presumably from sources in the pituitary (Imura et al., *Ann. Rev. Physiol. 43*: 265—278 (1981)), adrenal medulla (Viveros et al, *Adv. Biochem. Psychopharmacol. 22*: 191—204 (1980)), heart (Lang et al., *Life Sci. 32:* 399—406 (1983)), and gut (Elde et al., *Neuroscience 1:* 349—357 (1976); Polak et al., *Lancet 1*: 972—974 (1977); Alumets et al., *Histochem 56*: 187—196 (1978)).

A prevailing theory is that most actions of opioid drugs are within the central nervous system (that is, inside the brain or spinal cord). However, evidence that endogenous opioid peptides appear to condition the sensitivity of the peripheral nerves to stimuli that affect heart rate and blood pressure has been found,

and it is believed that circulating opioid peptides, under normal conditions, are operating to control the sensitivity of these peripheral sites of the automatic nervous system to such endogenous substances.

Endogenous opioid peptides can be grouped into three classes: β-endorphin and certain related compounds; the enkephalins, which are the smallest opioid peptides; and dynorphin, α-neo-dynorphin and their related peptides. Of the three types, β-endorphin seems to be the one most closely associated with morphine-like effects. Administration of this peptide intracerebroventricularly (i.c.v.) induces analgesia with both tolerance and physical dependence developing after prolonged treatment; moreover, cross-tolerance and cross-dependence are observed in relationship to morphine. In contrast, administration of the natural enkephalins, leucine-(leu) and methionine-(met) enkephalin has been reported to have very weak or no analgesic activity when given i.c.v.

Dynorphin was first isolated from pituitary glands, and the sequence of its first 13 N-terminal amino acids determined; this fragment has been synthesized and its properties studied along with that of the natural compound's full 17 amino acid sequence. The first 13 amino acids of dynorphin, or dynorphin(1—13), have the sequence:

TYR-GLY-GLY-PHE-LEU-ARG-ARG-ILE-ARG-PRO-LYS-LEU-LYS.
1      2      3      4      5      6      7      8      9      10      11      12      13

The N-terminal end contains Leu-enkephalin (those amino acids numbered 1—5), followed by the C-terminal extension (those amino acids numbered 6—13). The inclusion of Leu-enkephalin has been believed to be necessary as a biological "homing device" for activity, and the length of extension beyond Leu-enkephalin has been believed to be critical for its potency.

Dynorphin has little or no analgesic potency in mice. While this lack of effect was originally acribed to rapid degradation of dynorphin in the brain, it has been shown that dynorphin exhibited other pharmacological effects, indicating it should remain intact long enough to produce analgesia. Thus, U.S. Patent 4,361,553, inventors Loh et al., issued November 30, 1982, discloses that although dynorphin inhibits, or antagonizes, the analgesic response to both morphine and β-endorphin it has the opposite effect in tolerant animals. That is, dynorphin potentiates the analgetic effect of both morphine and β-endorphin in morphine-tolerant animals. Dynorphin thus behaves as neither a classical agonist nor an antagonist.

It has recently been reported that dynorphin (1—10) amide does not antagonize narcotic analgesics in naive animals (as does dynorphin (1—17) and dynorphin (1—13)), although it does potentiate the analgesic effect in tolerant hosts. Woo, et al., *Life Sciences 31*:1817—1882 (1982).

By contrast to the *in vivo* opioid properties of an opioid peptide such as dynorphin, naloxone (17 - allyl - 4,5α - epoxy - 3,14 - dihydroxymorphinan - 6 - one) behaves as a "classic" narcotic antagonist. Other non-peptide narcotics include naltrexone, nalorphine, diprenorphine, lavallorphan, pentazocine, metazocine, cyclazocine, and etazocine.

The present invention provides compositions for treating patients suffering from cerebral ischemia by administration of an opioid peptide. Suitable opioid peptides in accordance with the present invention include dynorphin, dynorphin analogs, and dynorphin amide analogs.

Preferred opioid peptides for practice of the present invention are those polypeptides having the amino acid sequence

TYR-GLY-GLY-PHE-LEU-ARG-ARG-AA$^8$-AA$^9$-AA$^{10}$-(AA$^{11}$)$_w$,

wherein AA$^8$ is TYR, ILU, LEU or LYS, AA$^9$ is ARG or PRO, AA$^{10}$ is PRO or LYS, AA$^{11}$ is LYS, LYS-LEU or LYS-LEU-LYS, w is 0 or 1, and with the polypeptide being in acid or amidated form. Two particularly preferred embodiments for practice of the present invention are dynorphin (1—13) and dynorphin (1—10) amide.

Preparation of suitable dynorphin and dynorphin-related peptides for practice of the present invention may be by methods and apparatus known to the art for peptide synthesis, with Example I, below (preparation of dynorphin (1—10) amide) being illustrative.

Example I

Dynorphin (1—10)-NH$_2$ was synthesized on a solid support of Boc-Pro-BHA (Benzyhydrylamine) resin (2mM/4.5 g of resin). With the Merrifield procedure on a Peninsula manual solid-phase peptide synthesizer, the corresponding Boc-protected amino acids were added respectively onto the Boc-Pro-BHA resin: Arg(Tos), Ile, Arg(Tos), Arg(Tos), Leu, Phe, Gly, Gly and Tyr(o-Br-Z). A 5.0 molar excess of each protected amino acid was used. The success of the coupling reaction was monitored by the semi-quantitative ninhydrin test. The following steps were employed to couple the Boc-protected amino acid to Boc-Pro-BHA resin:

1) Washing with CH$_2$Cl$_2$ (3×100 ml)
2) Prewashing with 33% TFA in CH$_2$Cl$_2$ with 1% indole (1×100 ml)
3) Deprotection with 33% TFA in CH$_2$Cl$_2$ with 1% indole (1×100 ml), 20 min.
4) Washing with CH$_2$Cl$_2$ (1×100 ml)

4

5) Washing with EtOH (1×100 ml)

6) Washing with CH$_2$Cl$_2$ (2×100 ml)

7) Prewashing with 10% Et$_3$N in CH$_2$Cl$_2$ (1×100 ml)

8) Neutralization with 10% Et$_3$N in CH$_2$Cl$_2$ (1×100 ml), 10 min.

9) Washing with CH$_2$Cl$_2$ (3×100 ml)

10) Protected amino acid (5.0 molar excess) in DMF (10 ml) and CH$_2$Cl$_2$ (50 ml) was added

11) DCC in CH$_2$Cl$_2$ (0.5 M, 20 ml) was added and the reaction time was up to three hours

12) Washing with CH$_2$Cl$_2$ (3×100 ml).

The resulting protected Boc-Tyr(O-Br-Z)-Gly-Gly-Phe-Leu-Arg(Tos)-Arg(Tos)-Ile-Arg(Tos)-Pro-BHA resin was washed well with 33% TFA in CH$_2$Cl$_2$, CH$_2$Cl$_2$ and MeOH respectively. After drying *in vacuo* overnight, the peptide resin was cleaved by HF (30 ml/g of resin) in the presence of anisole (3 ml/g of resin) for one hour at 0°C. The reaction mixture was dried *in vacuo* and washed with anhydrous ether. The desired peptide was dissolved in 10% HOAc and the resin was filtered off. The filtrate was lyophilized to give crude dynorphin(1—10)-NH$_2$. This peptide was purified by partition chromatography using n-BuOH:pyridine:H$_2$O (11:5:3) as eluting solvent and CM ion-exchange chromatography to afford the pure dynorphin(1—10)-NH$_2$.

It is believed that factors affecting response to therapy for cerebral ischemia in accordance with the present invention include the dosage, the route of administration, and duration of therapy.

However, blood pressure does not appear to be a factor affecting response to therapy for cerebral ischemia in accordance with the present invention, as blood pressure monitoring during practice of the invention has shown no changes in cardiac output, systemic blood pressure or cerebral blood flow.

In treating patients suffering from acute focal cerebral ischemia in accordance with the present invention, therapy is initiated by administering a dose of suitable opioid peptide and the preferably continued by administering subsequent doses.

The initial dose may be from 1.0 µg/kg of patient's weight to 10 mg/kg of patient's weight, more preferably 100 µg/kg of patient's weight, and can be delivered by various means known to the art, such as by intravenous injection ("I.V."). Subsequent doses may also be delivered by various means known to the art, such as by injections or through topical applications in conjunction with a drug carrier such as dimethyl sulfoxide or Azone® (available from Nelson Laboratories). However, it is preferred that the subsequent doses be delivered substantially continuously for as long as the patient is in a life threatening situation, or until the patient's condition stabilizes, and be at a rate between about 0.01 µg/h to about 100 µg/h. For example, continuous infusion may be by use of an implanted mini-pump, or by I.V. When the patient's condition stabilizes, then the doses may be gradually reduced, or titrated. Depending upon the mode of administration, the opioid peptide may be formulated with a wide variety of physiologically acceptable carriers, such as aqueous saline and phosphate buffered saline, and may include physiologically acceptable excipients, such as glucose or mannitol. Conventional methods may be used for preparing these compositions for administration.

The following experimental methods, materials and results are described for purposes of illustrating the present invention.

Experimental

On the basis of a random number table, adult male cats were assigned to one of six groups to be treated with: 1) saline (12 cats), 2) naloxone (13 cats), 3) naltrexone (10 cats), 4) diprenorphine (13 cats), 5) dinorphin (1—13) (10 cats), and 6) dynorphin (1—10) amide (5 cats). The cats were sedated with 50 mg of ketamine administered intramuscularly. Anesthesia was induced by admixture of halothane, nitrous oxide, and oxygen administered by mask. The trachea was then intubated but the cat was allowed to breath spontaneously. One million units of penicillin G were then administered intramuscularly and the cats were placed in a stereotactic apparatus. Transorbital occlusion of the right middle cerebral artery (MCA) was performed using the technique in cats first described by O'Brien and Waltz. ("Transorbital approach for occluding the middle cerebral artery without craniectomy," *Stroke 4*: 201—206 (1973).)

An incision was made in the supraorbital region using aseptic technique, and dissection was performed in a subperiosteal plane along the roof of the orbit. The globe was incised and the contents removed. The ciliary arteries and ophthalmic vessels were coagulated with bipolar coagulating forceps under magnification of the surgical microscope. Straight and curved microscissors were used to complete the orbital dissection and the orbital contents were evacuated. A microsurgical drill was used to remove the optic strut, thus enlarging the optic foramen. The dura was incised and the carotid bifurcation was exposed. An arachnoid dissection was formed to free the internal carotid, middle cerebral, posterior communication, and anterior cerebral arteries. The segment of the MCA proximal to the lenticulostriate arteries (MI) was coagulated with the bipolar forceps and transected with microscissors. The orbit was irrigated and filled with dental cement to prevent leakage of CSF. The wound was sutured closed and colloidin spray was applied as a dressing.

A small incision was made in the midline in the lumbar region and a subcutaneous pocket was created for the later placement of an osmotic pump that was designed to deliver drug at a constant volume. This incision was sutured loosely using a running stitch. The cat was allowed to awaken and was examined 6 hours following MCA occlusion. Neurologic function was assessed independently by two individuals who were unaware of the experimental protocol.

In a blind study, the cats then received an intraperitoneal injection of one of the following solutions: 2 ml of sterile normal saline; 2 ml of a 10 mg/kg solution of naloxone dissolved in sterile normal saline; 2 ml of a solution containing 500 µg of diphrenorphine dissolved in sterile normal saline; 2 ml of a solution containing 10 mg/kg of naltrexone dissolved in sterile normal saline; 2 ml of a solution containing 10 mg/kg of dynorphin (1—13) dissolved in sterile normal saline; and, 2 ml of a solution containing 10 mg/kg of dynorphin (1—10) amide dissolved in sterile, normal saline. A second neurologic assessment was performed 20 minutes later.

The cats were then sedated with 50 mg of ketamine administered intramuscularly and using sterile technique, an osmotic pump (available from ALZA Corp., Palo Alto, CA) was implanted in the previously created subcutaneous pocket in the lumbar region to deliver either saline at 10 µl/h, diprenorphine at 100 µg/h, naloxone at 5 mg/kg/h, naltrexone at 1 mg/kg/h, 50 µg/h dynorphin (1—13) or 50 µg/h dynorphin (1—10) amide. Again, the investigators were not aware as to which treatment was being administered.

Neurologic assessments were performed daily for as long as the cats were alive, or until 7 days had elapsed. One million units of penicillin G were administered intramuscularly daily and subcutaneous injections of sufficient lactated Ringer's solution provided adequate daily fluid maintenance. Once a cat began to eat or drink, subcutaneous fluids were discontinued. If a cat was found dead, a craniectomy was performed, the brain removed, and a coronal section was made at the level of optic chiasm. After 7 days had elapsed the surviving cats were sacrificed.

The coronal sections were incubated in a 2% solution of 2,3,5 triphenyltetrazolium chloride (TTC) for 25 minutes. TTC has been used extensively to demonstrate the presence and extent of acute myocardial infarcts, and gives a vivid indication of cerebral infarction in the acute state. The reaction product of TTC and viable tissue is a deep red formazan that deeply stains normal gray matter, whereas normal white matter stains with lesser intensity. Infarcated tissue does not stain.

Color slides were made of the stained brains. A neuropathologist who was unaware of the experimental protocol made tracings of the entire affected hemisphere and infarcted area from projected images of the slide. Using a digitizer, the percentage of infarcted tissue relative to the entire hemisphere was calculated for both sections in each cat; this defined the infarct size.

Table I, below, illustrates the mortality for each group as a function of time following the cerebral artery occlusions.

TABLE I

|  | less than 24 hours | 24—28 hours | 48—72 hours | lived up to 7 days |
|---|---|---|---|---|
| Saline | 100% | 0% | 0% | 0% |
| Diprenorphine | 84% | 8% | 8% | 0% |
| Naloxone | 54% | 15% | 0% | 31% |
| Naltrexone | 40% | 10% | 10% | 40% |
| Dynorphin (1—10) amide | 80% | 0% | 0% | 20% |
| Dynorphin (1—13) | 20% | 20% | 0% | 60% |

As illustrated by the data of Table I above, all cats of the control, or saline group died in less than 24 hours. The administration of diprenorphine had little effect in prolonging survival. Both naloxone and naltrexone prolonged survival. Dynorphin (1—13) prolonged survival best of all the groups. Although the mortality rate results for dynorphin (1—10) amide in prolonging survival appear less impressive than for naloxone, naltrexone and dynorphin (1—13), the conditions of survival were of high quality and clinically quite significant.

The infarct size was not altered by any treatment administered and there was substantial similarity in the results among groups. That is, there was not statistically significant difference in infarct size among the groups.

**Claims**

1. Use of an opioid peptide for the production of an agent against cerebral ischemia or an agent for prolonging survival of a patient suffering from acute focal cerebral ischemia, the opioid peptide having the amino acid sequence

TYR-GLY-GLY-PHE-LEU-ARG-ARG-AA$^8$-AA$^9$-AA$^{10}$-(AA$^{11}$)$_w$,

wherein AA[8] is TYR, ILE, LEU or LYS, AA[9] is ARG or PRO, AA[10] is PRO or LYS, AA[11] is LYS, LYS-LEU or LYS-LEU-LYS, w is 0 or 1, and being in acid or amidated form.

2. Use according to claim 1 wherein the opioid peptide is dynorphin (1—13) or dynorphin (1—10) amide.

3. Use according to claim 1 or claim 2 wherein said agent is a pharmaceutical composition comprising the said opioid peptide and a pharmaceutically acceptable carrier.

4. Use according to claim 3 wherein the composition contains the said opioid peptide or a plurality of such peptides as the only active ingredient(s).

5. Use according to any one of the preceding claims wherein the agent contains, as a single dose, a total amount of said opioid peptide(s) in the range 70 µg to 700 mg.

**Patentansprüche**

1. Verwendung eines Opioid-Peptides für die Herstellung eines Mittels gegen zerebrale Ischämie oder eines Mittels zur Verlängerung der Lebensdauer eines Patienten, der an akuter fokaler zerebraler Ischämie leidet, welches Opioid-Peptid die Aminosäuresequenz

$$\text{TYR-GLY-GLY-PHE-LEU-ARG-ARG-AA}^8\text{-AA}^9\text{-AA}^{10}\text{-(AA}^{11})_w$$

aufweist, worin AA[8] TYR, ILE, LEU oder LYS bedeutet, AA[9] für ARG oder PRO steht, AA[10] PRO oder LYS ist, AA[11] LYS, LYS-LEU oder LYS-LEU-LYS darstellt, w 0 oder 1 bedeutet, und in Säureform oder amidierter Form vorliegt.

2. Verwendung nach Anspruch 1, worin das Opioid-peptid (Dynorphin (1—13) oder Dynorphin (1—10) amid ist.

3. Verwendung nach Anspruch 1 oder 2, worin das genannte Mittel eine pharmazeutische Zusammensetzung ist, die das genannte Opioid-Peptid und ein pharmazeutisch verträgliches Trägermittel enthält.

4. Verwendung nach Anspruch 3, worin die Zusammensetzung das genannte Opioid-Peptid oder eine Mehrzahl solcher Peptide als einzige(n) Wirkstoff(e) enthält.

5. Verwendung nach einem der vorhergehenden Ansprüche, worin das Mittel als einzige Dosis eine Gesamtmenge des(der) genannte(n) Opioid-Peptide(s) im Bereich von 70 µg bis 700 mg enthält.

**Revendications**

1. Utilisation d'un peptide opioïde pour le production d'un agent contre l'ischémie cérébrale ou d'un agent pour prolonger la survie d'un patient souffrant d'ischémie cérébrale focale aiguë, le peptide opioïde ayant la séquence d'acides aminés

$$\text{TYR-GLY-GLY-PHE-LEU-ARG-ARG-AA}^8\text{-AA}^9\text{-AA}^{10}\text{-(AA}^{11})_w,$$

où AA[8] est TYR, ILE, LEU ou LYS, AA[9] est ARG ou PRO, AA[10] est PRO ou LYS, AA[11] est LYS, LYS-LEU ou LYS-LEU-LYS, w est 0 ou 1 et qui est sous une forme d'acide ou d'amide.

2. Utilisation selon la revendication 1 où le peptide opioïde est dynorphine (1—13) ou dynorphine (1—10) amide.

3. Utilisation selon la revendication 1 ou la revendication 2 où ledit agent est une composition pharmaceutique comprenant ledit peptide opioïde et un véhicule acceptable en pharmacie.

4. Utilisation selon la revendication 3 où la composition contient ledit peptide opioïde ou un certain nombre de ces peptides en tant que seul(s) ingrédient(s) actif(s).

5. Utilisation selon l'une quelconque des revendications précédentes où l'agent contient, sous la forme d'une seule dose, une quantité totale dudit ou desdits peptides opioïdes comprise entre 70 µg et 700 mg.